# EUROPÄISCHE PATENTSCHRIFT

(11) **EP 4 117 747 B1**
(45) Veröffentlichungstag und Bekanntmachung des Hinweises auf die Patenterteilung: **15.05.2024**
(21) Anmeldenummer: 21707922.7
(22) Anmeldetag: 22.02.2021
(51) Int. Cl.: A61M 1/16

(54) **KONNEKTIONSELEMENT UND KONNEKTIONSSYSTEM FÜR EINE BLUTBEHANDLUNGSVORRICHTUNG**
CONNECTOR, AND CONNECTOR SYSTEM FOR A BLOOD TREATMENT DEVICE
CONNECTEUR, AND SYSTÈME DE CONNECTION POUR UN DISPOSITIF DE TRAITEMENT DE SANG

(30) Priorität: 11.03.2020 DE 102020106590
(43) Veröffentlichungstag der Anmeldung: 18.01.2023
(73) Patentinhaber: Fresenius Medical Care Deutschland GmbH, 61352 Bad Homburg (DE)
(72) Erfinder: BREHM, Winfried, 97461 Hofheim (DE); STERZER, Rafael, 97422 Schweinfurt (DE); SEIT, Paul, 97421 Schweinfurt (DE)
(74) Vertreter: Rau, Jenspeter
(86) Internationale Anmeldenummer: PCT/EP2021/054272
(87) Internationale Veröffentlichungsnummer: WO 2021/180450

(56) Entgegenhaltungen:
- EP-A1- 2 682 608
- WO-A1-00/16916
- WO-A1-2019/086321
- WO-A2-2013/135389
- DE-A1-102009 024 575
- DE-B4-102013 001 438

## Beschreibung

### Technisches Gebiet

Die vorliegende Erfindung betrifft ein Konnektionselement für eine Blutbehandlungsvorrichtung, insbesondere eine Dialysemaschine, ein Konnektionssystem für eine Blutbehandlungsvorrichtung, insbesondere eine Dialysemaschine, sowie ein Konnektionsverfahren zur Konnektion eines Trockenkonzentratbehälters.

### Hintergrund

Unter dem Begriff der Blutbehandlungsvorrichtung ist unter anderem eine Dialysemaschine zu verstehen. Dialysemaschinen finden häufig in Dialysezentren zur Behandlung einer chronischen Nierenerkrankung Anwendung.

Während der Dialyse strömt durch die Blutkammer des Dialysators kontinuierlich Blut des Patienten, während durch die Dialysierflüssigkeitskammer fortlaufend Dialysierflüssigkeit fließt. Zur Herstellung von Dialysierflüssigkeit können vorgefertigte Dialysierflüssigkeitskonzentrate verwendet werden, die in den Dialysevorrichtungen mit Wasser verdünnt werden. In Dialysezentren werden Dialysierflüssigkeitskonzentrate entweder als vorgefertigtes Produkt in Kanistern, Beuteln oder Kartuschen zur Verfügung gestellt oder über ein Ringleitungssystem aus einem zentralen Tank bereitgestellt.

Zentral zur Verfügung gestellte Dialysierflüssigkeitskonzentrate sind für den Anwender einfach zu handhaben, sie haben aber den Nachteil, dass die Dialysierflüssigkeit nicht individuell auf die Bedürfnisse des Patienten abgestimmt werden kann. Dezentral bereitgestellte Konzentrate erlauben zwar eine individuelle Anpassung der Dialysierflüssigkeit an den Patienten, sie müssen aber für jede einzelne Dialysebehandlung an die Dialysevorrichtung gebracht werden.

In der Dialyse finden für die Herstellung eines flüssigen Dialysekonzentrats Beutel oder Kartuschen Verwendung, die mit einem pulverförmigen Dialysierflüssigkeitskonzentrat befüllt sind. Die Konzentratbeutel oder -kartuschen enthalten eine Menge an pulverförmigen Dialysierflüssigkeitskonzentrat, die für eine einzige Dialysebehandlung ausreichend ist. Die Beutel oder Kartuschen sind mit Bikarbonat befüllt. Handelsübliche Bikarbonatbeutel enthalten 650 bis 950 g Natriumbicarbonat. Aus dem pulverförmigen Bikarbonat-Konzentrat wird zunächst ein flüssiges Bikarbonat-Konzentrat hergestellt. Für die Herstellung der Dialysierflüssigkeit ist ein weiteres Säurekonzentrat erforderlich, das in einem Kanister oder mit einer zentralen Versorgung bereitgestellt wird. Bikarbonat-Konzentrat und Säurekonzentrat werden dann mit Wasser zu der fertigen Dialysierflüssigkeit gemischt.

Die Dialysemaschine weist ferner einen Wasseranschluss auf, über welchen Reinwasser zugeführt wird. In der Dialysemaschine erfolgt anschließend die gewünschte Mischung von Reinwasser mit dem entsprechenden Konzentrat.

Die Bereitstellung des flüssigen Konzentrats über Konzentratbehälter bringt verschiedene Herausforderungen mit sich, da die Konzentratbehälter schwer und damit für das Pflegepersonal aufwendig in der Handhabung sind. Zudem müssen zur Bereitstellung des Konzentrats Ansaugstäbe der Dialysemaschine in die Konzentratbehälter eingebracht werden, welche nach einer Behandlung zur Reinigung wieder in die Dialysemaschine gesteckt werden. Diese Handhabung der Konzentratbehälter ist damit zeitintensiv und kann zu einer Verlängerung der Pausen zwischen den einzelnen Behandlungen führen.

Alternativ kann das Konzentrat auch über eine Zentralversorgung und einen entsprechenden Zentralversorgungsanschluss an der Dialysemaschine zugeführt werden. Dies setzt aber eine entsprechende Ausstattung der Klinik voraus, und kann bezüglich der Flexibilität des zur Verfügung stehenden Konzentrats und dem Wartungsaufwand der hierfür betriebenen Leitungen Nachteile mit sich bringen.

Aus der WO 00/16916 A1 ist eine Verbindungseinrichtung zur Fluidverbindung mit Abfuhrleitung und Reinigungsstrategie offenbart. Zur Realisierung der Reinigungsstrategie ist keine Abfuhrleitung vorgesehen, welche einen Teilabschnitt um eine Einlassleitung hat. Die Verbindungseinrichtung weist vielmehr eine Einlassleitung sowie eine Auslassleitung auf, welche stromaufwärts in Fluidverbindung mit einem Abführkanal steht.

WO 2019/086321 A1 offenbart eine Verbindungseinrichtung zur Fluidverbindung mit Reinigungsstrategie.

DE 10 2009 024575 A1 offenbart eine Verbindungseinrichtung zur Fluidverbindung wenigstens eines ersten mit einem zweiten fluidführenden medizintechnischen System mit wenigstens einem ersten Rohrstück des ersten Systems, welches vorgesehen ist, mit einem zweiten Rohrstück des zweiten Systems verbunden zu werden.

DE 10 2013 001438 B4 betrifft eine medizinische Vorrichtung mit einer Buchsen-Einheit zum Anschluss einer Stecker-Einheit einer Vorrichtung zur Bereitstellung von medizinischen Flüssigkeiten, wobei die medizinische Vorrichtung insbesondere eine extrakorporale Blutbehandlungsvorrichtung, beispielsweise extrakorporale Dialyse Vorrichtung oder Vorrichtung zur Peritonealdialyse, und die medizinische Flüssigkeit insbesondere eine Dialyseflüssigkeit ist.

WO 2013/135389 A2 betrifft eine medizinische Behandlungsvorrichtung, insbesondere extrakorporale Blutbehandlungsvorrichtung, mit einer Buchsen-Einheit zum Anschluss einer Stecker-Einheit einer Vorrichtung zur Bereitstellung einer medizinischen Flüssigkeit für die medizinische Behandlungsvorrichtung.

EP 2 682 608 A1 betrifft einen Multikonnektor zur Anbringung eines Schlauchsegments und zu- und abführenden Leitungen eines extrakorporalen Blutkreislaufs an der Schlauchrollenpumpe eines medizinischen Geräts. Über die geometrische Form derartiger Multikonnektoren kann auch ihre Präsenz in der Pumpe detektiert werden, indem beispielsweise ein zylindrischer Abschnitt des Multikonnektors während des Einsetzvorgangs einen Stößel betätigt, dessen axiale Position über eine Lichtschranke abgefragt wird.

Aus der EP 1 344 550 A1 ist ein Konnektor zur Verbindung eines Konzentratbeutels, welcher mit Trockenkonzentrat gefüllt ist und an einer Dialysemaschine eingehängt werden kann, bekannt. Auch bei dieser Art der Konzentratversorgung sind bestimmte Schritte zwischen den Behandlungen notwendig. So muss ein am Konzentratbeutel angebrachter Kontaminationsschutz manuell entfernt, der Konzentratbeutel in die entsprechende Aufnahme an der Dialysemaschine eingesetzt, das heißt konnektiert, werden, und schließlich mit dem Dialysatkreislauf verbunden werden.

Die Konnektion des Konzentratbeutels erfolgt im Stand der Technik zumeist von unten. Das heißt, der Konzentratbeutel wird auf Anschlusstüllen aufgesteckt. Nach der Behandlung wird der Konzentratbeutel von den Anschlusstüllen gehoben. Dabei kann Restflüssigkeit, welche noch in den Anschlussstellen des Konzentratbeutels vorhanden ist, leicht auf den Boden tropfen.

Dabei kann Restflüssigkeit der Bicarbonatlösung aber auch zu Ablagerungen an den Anschlusstüllen der Dialysemaschine führen. Dies wird durch Kalzium- und Magnesiumsalze verursacht. Durch die Ausbildung der nach unten geöffneten Anschlussstellen kann diese Restflüssigkeit in die Anschlussstellen, der Dialysemaschine tropfen und dort die Ablagerungen verursachen. Diese können unter anderem zu Beschädigungen an Dichtringe führen.

Wie beschrieben, muss der Konzentratbeutel nach einer Behandlung von dem Dialysatkreislauf dekonnektiert und entfernt werden. Erst nach der Entfernung kann ein Reinigungsprogramm an der Dialysemaschine gestartet werden. Auch die Oberflächenreinigung der Dialysemaschine kann erst nach dem Entfernen des Konzentratbeutels von dem Pflegepersonal durchgeführt werden.

Neben hohen Sicherheitsanforderungen spielt bei der Dialysebehandlung, insbesondere in Dialysezentren, der zeitliche Aspekt eine besondere Rolle.

Da ausreichend Zeit für die tatsächliche Behandlung zur Verfügung stehen soll, ist insbesondere die Zeit zwischen den Behandlungen einer ständigen Zeitoptimierung unterworfen. Wie oben beschrieben, nimmt zwischen den Behandlungen neben der Vorbereitung des Patienten, auch die Vorbereitung der Dialysemaschine für die nächste Behandlung, durch Schritte, wie der Bereitstellung der benötigten Konzentratbehälter oder der Desinfizierung der Dialysemaschine, Zeit in Anspruch.

Der vorliegenden Anmeldung liegt damit die Aufgabe zu Grunde ein Konnektion eines Konzentratbeutels an einer Blutbehandlungsvorrichtung zu ermöglichen, die eine verbesserte Handhabung und/oder eine Zeiteinsparung für den Nutzer erlaubt, sowie gleichzeitig die Sicherheit des Patienten gewährleistet, indem unter anderem eine einfache Reinigung möglich ist.

### Zusammenfassung der Erfindung

Die der Erfindung zugrunde liegende Aufgabe wird durch ein Konnektionselement für eine Blutbehandlungsvorrichtung gemäß Anspruch 1, durch ein Konnektionssystem für eine Blutbehandlungsvorrichtung nach Anspruch 8, ein Konnektionsverfahren nach Anspruch 11 sowie die Verwendung des Konnektionselements bei einer Blutbehandlungsvorrichtung nach Anspruch 15 gelöst. Vorteilhafte Weiterentwicklungen und Ausführungsformen sind Gegenstand der abhängigen Ansprüche. Erfindungsgemäß ist ein Konnektionselement ausgebildet, das eine Einlassleitung, zum Einlassen eines Fluids in das Konnektionselement, eine Auslassleitung, zum Auslassen eines Fluids aus dem Konnektionselement aufweist. Weiter kann zumindest eine erste Abfuhrleitung, zum Abführen eines Fluids aus dem Konnektionselement, ausgebildet sein. Die erste Abfuhrleitung kann in einem Teilabschnitt um die Einlassleitung herum ausgebildet sein. Die Einlassleitung und die erste Abfuhrleitung können zu einem Endabschnitt des Konnektionselements geöffnet sein und der Endabschnitt kann fluidisch mit dem Teilabschnitt verbunden sein.

Dabei kann die erste Abfuhrleitung mit der Einlassleitung und/oder der Auslassleitung identisch sein. Dabei kann an einem einer Dialysemaschine, beziehungsweise einem Dialysemaschinenanschluss, gegenüberliegenden Ende der Einlassleitung und/oder Auslassleitung durch Bereitstellen einer Reinigungsvorrichtung, beispielsweise eines Sacklochs, in welches jeweils die Einlassleitung und Auslassleitung eingebracht werden kann, ein Aufstauen der Reinigungsflüssigkeit erreicht werden. Nach einer gewissen Zeit kann die Reinigungsflüssigkeit wieder durch die Einlassleitung und/oder Auslassleitung abgeführt werden. Alternativ kann zwischen der Einlassleitung und der Auslassleitung ein Verbindungskanal in der Reinigungsvorrichtung ausgebildet sein. Dabei kann die Reinigungsflüssigkeit über eine der Einlassleitung und/oder Auslassleitung zugeführt werden. Die andere der beiden Leitungen ist, beispielsweise durch ein Ventil, abgesperrt. Damit wird die Reinigungsflüssigkeit in beiden Einlass- und Auslassleitungen aufgestaut. Auch hier kann nach einer bestimmten Zeit ein Abführen über eine oder beide Einlassleitung und/oder Auslassleitung erfolgen.

Die Abfuhrleitung kann ebenso um die Einlassleitung und/oder Auslassleitung ausgebildet sein und kann mit einer in der Reinigungsvorrichtung ausgebildeten Anschlussleitung verbunden sein, so dass Fluid über die Abfuhrleitung die Einlass- und/oder Auslassleitung umspült und weiter über die Anschlussleitung abgeführt wird.

Die Einlassleitung und Auslassleitung können in ihrem Innendurchmesser zumindest teilweise zylinderförmig ausgebildet sein. Ebenso kann die Innenkontur konisch zulaufend oder stufenartig ausgebildet sein.

Nach einer Weiterbildung kann das Konnektionselement weiter eine zweite Abfuhrleitung, zum Abführen eines Fluids aus dem Konnektionselement, aufweisen. Dabei kann die zweite Abfuhrleitung in einem zweiten Teilabschnitt um die Auslassleitung herum ausgebildet sein. Die Auslassleitung und die Abfuhrleitung können zu einem Endabschnitt des Konnektionselement geöffnet sein und der Endabschnitt kann fluidisch mit dem Teilabschnitt verbunden sein.

Nach einer Weiterbildung des Konnektionselements kann die Einlassleitung und/oder Auslassleitung und/oder die erste und zweite Abfuhrleitung derart in dem Konnektionselement angeordnet sein, dass eine Umlenkung der Fluidrichtung in dem Konnektionselement, vorzugsweise um 120 bis 70 Grad, noch bevorzugter von 110 bis 80 Grad, besonders bevorzugt von im Wesentlichen 90 Grad, erreichbar ist.

Nach einer Weiterbildung kann das Konnektionselement weiter einen Mittelabschnitt, und einen ersten Fortsatz, in dem die erste Abfuhrleitung um die Einlassleitung ausgebildet ist, welcher sich von einem ersten Ende des Mittelabschnitts in einer ersten Richtung von dem Mittelabschnitt erstreckt, einen zweiten Fortsatz, in dem die zweite Abfuhrleitung um die Auslassleitung ausgebildet ist, welcher sich von einem zweiten, dem ersten gegenüberliegenden, Ende des Mittelabschnitts in der ersten Richtung, vorzugsweise parallel zu dem ersten Fortsatz von dem Mittelabschnitt erstreckt, auf.

Der erste Fortsatz und/oder der zweite Fortsatz können dabei zylinderförmig ausgebildet sein. Ebenso können der erste und/oder zweite Fortsatz konisch oder stufenartig sowie teleskopartig ausfahrbar ausgebildet sein.

Der erste und/oder zweite Fortsatz können sich von einem Mittelabschnitt in einer ersten Richtung, vorzugsweise im Wesentlichen senkrecht, von dem Mittelabschnitt erstrecken.

Nach einer Weiterbildung kann das Konnektionselement weiter zumindest zwei, vorzugsweise drei oder vier, noch bevorzugter vier, vorzugsweise zueinander parallel verlaufende, vorzugsweise zylinderförmige Vorsprünge, aufweisen. Dabei können sich die Einlassleitung und die erste Abfuhrleitung in je einem Vorsprung von dem ersten Ende des Mittelabschnitts in einer zweiten, von der ersten verschiedenen Richtung, erstrecken. Zudem können sich die Auslassleitung und die zweite Abfuhrleitung in je einem Vorsprung von dem zweiten Ende des Mittelabschnitts in der zweiten Richtung erstrecken. Der zumindest eine Vorsprung der zumindest einen Abfuhrleitung kann sich in einer Richtung von dem Konnektionselement erstrecken, die verschieden von der Richtung ist, in welche sich der Vorsprung der Einlassleitung und/oder Auslassleitung erstreckt. Zudem können sich die Einlassleitung und Auslassleitung zu verschiedenen Richtungen erstrecken.

Nach einer Weiterbildung kann das Konnektionselement weiter Führungselemente zur verfahrbaren, vorzugsweise linear verschiebbaren, Verbindung mit einer Führungsvorrichtung, aufweisen.

Die Führungselemente können dabei integral mit dem Konnektionselement ausgebildet sein oder an diesem durch Verschrauben, Verkleben oder Verschweißen angebracht werden. Die Führungselemente können Schienenelemente sein, welche in Schienen eingesetzt und in diesen linear verschiebbar angeordnet werden können.

Nach einer Weiterbildung kann das Konnektionselement weiter ein Positionierungselement aufweisen. Das Positionierungselement kann in einer dritten, von der ersten und zweiten verschiedenen Richtung, von dem Mittelabschnitt hervorstehen. Mit anderen Worten kann das Positionierungselement in Richtung der Führungselemente von dem Konnektionselement hervorstehen. Das Positionierungselement kann ein integral an dem Konnektionselement ausgebildeter Fortsatz, beispielsweise ein rechteckiger oder zylinderförmiger Fortsatz sein. Das Positionierungselement kann derart von dem Konnektionselement hervorstehen, dass es bei einem linearen Verfahren des Konnektionselements mit einer Lichtschranke wechselwirken kann. Alternativ kann das Positionierungselement ein Hall-Sensor sein.

Erfindungsgemäß weist das Konnektionssystem ein Konnektionselement, vorzugsweise nach einem der vorhergenannten Aspekte, ein Gehäuse, in welchem das Konnektionselement bewegbar angeordnet ist, auf. Weiter kann das Konnektionssystem ein Reinigungselement aufweisen, welches, vorzugsweise bewegbar in dem Gehäuse angeordnet ist. Das Reinigungselement kann außerhalb des Gehäuses angeordnet sein. Weiter kann das Konnektionssystem zumindest eine erste Antriebseinheit, zum Verfahren des Konnektionselements und/oder des Reinigungselements, sowie eine Steuereinheit zur Ansteuerung der zumindest ersten Antriebseinheit, aufweisen. Dabei kann das Konnektionselement und/oder das Reinigungselement ebenso manuell verschiebbar ausgebildet sein.

Nach einer Weiterbildung kann das Konnektionssystem weiter Konnektionsführungen, welche in dem Gehäuse angeordnet sind, auf welchen das Konnektionselement zwischen mehreren, vorzugsweise drei, Positionen verfahrbar ist, aufweisen. Weiter können Reinigungsführungen, vorzugsweise in dem Gehäuse, angeordnet sein, auf welchen das Reinigungselement zwischen mehreren, vorzugsweise zwei, Positionen verfahrbar ist.

Nach einer Weiterbildung kann das Konnektionssystem weiter zumindest einen Positionssensor in dem Gehäuse, vorzugsweise eine Lichtschranke, mittels welcher die Position des Konnektionselements und/oder des Reinigungselements erfassbar ist, aufweisen.

Erfindungsgemäß weist ein Konnektionsverfahren zur Konnektion eines Trockenkonzentratbehälters die nachfolgenden Schritte auf. Dabei kann ein Gehäuse ein in dem Gehäuse auf einer ersten Geraden verfahrbaren Konnektionselement, vorzugsweise nach einem der vorhergegangenen Aspekte, aufweisen. Das Konnektionselement kann zwei Fortsätze aufweist, und eine, vorzugsweise in dem Gehäuse auf einer zweiten, zu der ersten Geraden im Wesentlichen senkrechten Geraden, verfahrbares Reinigungselement, in welchem ein erstes und ein zweites Leitungselement ausgebildet sind. Das Konnektionsverfahren kann die Schritte Verfahren des Konnektionselements auf der ersten Geraden zur Ausbildung einer Reinigungsstellung, wobei in der Reinigungsstellung der erste und zweite Fortsatz des Konnektionselement in Eingriff mit dem ersten und zweiten Leitungselement der Reinigungsvorrichtung sind, aufweisen.

Das erste und zweite Leitungselement können dabei Sackbohrungen, beziehungsweise Sachlöcher sein. Ebenso können die Leitungselemente Durchgangsbohrungen sein und/oder es kann eine Verbindungsleitung zwischen dem ersten und zweiten Leitungselement ausgebildet sein. Die Verfahrensschritte des Verfahrens des Konnektionselements und/oder der Reinigungsvorrichtung können dabei automatisch, beispielsweise durch eine Antriebseinheit oder durch manuelle Betätigung eines Pflegepersonals, durchgeführt werden. Ebenso kann auch nur die Reinigungsvorrichtung oder nur das Konnektionselement manuell betätigt werden.

Nach einer Weiterbildung kann das Konnektionsverfahren weiter ein Verfahren des Konnektionselements auf der ersten Geraden, Verfahren der Reinigungsvorrichtung auf der zweiten Geraden, Verfahren des Konnektionselements auf der ersten Geraden zur Ausbildung der Konnektionsstellung, wobei in dieser Konnektionsstellung, der erste und zweite Fortsatz aus dem Gehäuse hervorstehen, aufweisen.

Nach einer Weiterbildung kann das Konnektionsverfahren weiter ein Anbringen eines Behälters, vorzugsweise mit Trockenkonzentrat gefüllt, an dem Gehäuse, wenn das Konnektionselements in der Reinigungsstellung ist, ein Erkennen, ob ein Konzentratbehälter zur Konnektion bereitgestellt ist, ein Verfahren des Konnektionselements auf der ersten Geraden in die Konnektionsstellung, sowie ein Verbinden des Behälters mit dem ersten und zweiten Fortsatz des Konnektionselements, so dass eine fluidische Verbindung hergestellt wird, aufweisen.

Nach einer Weiterbildung kann das Konnektionsverfahren, wobei das Konnektionselement fluidisch mit einer Blutbehandlungsvorrichtung verbindbar ist, weiter ein Verfahren des Konnektionselements auf der ersten Geraden in einer ersten Richtung, ein Verfahren des Reinigungselements auf der zweiten Geraden, ein Verfahren des Konnektionselements auf der ersten Geraden ein einer der ersten Richtung entgegengesetzten Richtung zur Ausbildung der Reinigungsstellung, so dass der erste und zweite Fortsatz jeweils in dem ersten und zweiten Leitungselement der Reinigungsvorrichtung aufgenommen sind, sowie ein Reinigen des ersten und zweiten Fortsatzes in dem ersten und zweiten Leitungselements, aufweisen.

Erfindungsgemäß ist weiter eine Verwendung eines Konnektionselements nach einem der vorhergegangenen Aspekte bei einer Blutbehandlungsvorrichtung, vorgesehen.

Durch das Konnektionselement sowie das Konnektionssystem ist es möglich einen Konzentratbeutel leicht, das heißt mit wenigen Handhabungsschritten mit einer Blutbehandlungsvorrichtung, insbesondere einer Dialysemaschine, zu verbinden. Insbesondere kann ein Konzentratbeutel an der Blutbehandlungsvorrichtung angebracht werden, während noch ein Reinigungsschritt der Konnektionstüllen erfolgt. Durch die Ausbildung des Konnektionselements und Konnektionssystems ist es zudem möglich, die Sicherheit des Patienten zu gewährleisten.

Die vorstehend beschriebenen Merkmale und Funktionen der vorliegenden Erfindung sowie weitere Aspekte und Merkmale werden nachfolgend anhand einer detaillierten Beschreibung von bevorzugten Ausführungsformen unter Bezugnahme auf die beigefügten Figuren weiter beschrieben. In den Figuren sind gleiche Merkmale/Elemente und Merkmale/Elemente mit der gleichen Funktion mit den gleichen Bezugszeichen gekennzeichnet.

### Kurzbeschreibung der Zeichnungen

Hierbei zeigt:
- Fig. 1: eine perspektivische Prinzipdarstellung einer Konnektionselements nach einer ersten Ausführungsform;
- Fig. 2: eine Schnittansicht des Konnektionselements nach einer zweiten Ausführungsform;
- Fig. 3: eine Teilansicht eines Konnektionssystems einer ersten Ausführungsform;
- Fig. 4: eine Teilansicht des Konnektionssystems nach der ersten Ausführungsform;
- Fig. 5: eine Teilansicht des Konnektionssystems nach einer zweiten Ausführungsform;
- Fig. 6: eine Teilansicht der Reinigungsvorrichtung des Konnektionssystems;
- Fig. 7: eine perspektivische Ansicht des Konnektionssystems nach der ersten Ausführungsform;
- Fig. 8a,: b eine Prinzipdarstellung von Freiheitsgraden eines Konnektionsverfahrens nach einer ersten Ausführungsform;
- Fig. 9: eine Prinzipdarstellung von Freiheitsgraden eines Konnektionsverfahrens nach einer zweiten Ausführungsform;
- Fig. 10: eine Prinzipdarstellung von Freiheitsgraden eines Konnektionsverfahrens nach der zweiten Ausführungsform;
- Fig. 11a,: b eine Prinzipdarstellung von Freiheitsgraden eines Konnektionsverfahrens nach einer dritten Ausführungsform;
- Fig. 12a,: b eine Prinzipdarstellung von Freiheitsgraden eines Konnektionsverfahrens nach einer vierten Ausführungsform;
- Fig. 13: eine Prinzipdarstellung eines Verfahrensschritts zur Erkennung eines Konzentratbehälters;
- Fig. 14: eine Prinzipdarstellung eines Verfahrensschritts einer alternativen Erkennung eines Konzentratbehälters;
- Fig. 15: eine Prinzipdarstellung eines Verfahrensschritts einer weiteren Erkennung eines Konzentratbehälters; und
- Fig. 16: eine Prinzipdarstellung eines Verfahrensschritts einer anderen Erkennung eines Konzentratbehälters.

### Detaillierte Beschreibung eines Ausführungsbeispiels

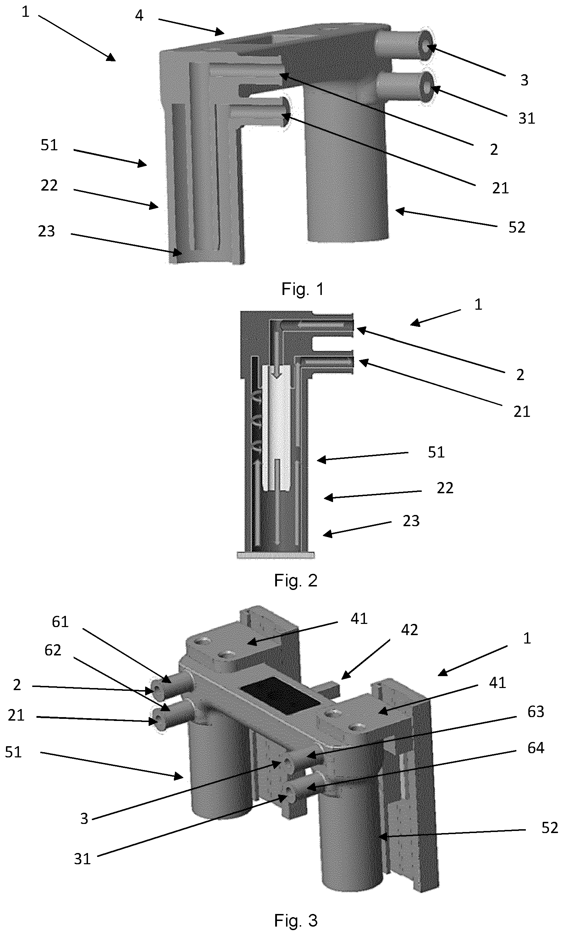

Bezugnehmend auf Fig. 1 wird nachstehend eine erste Ausführungsform erläutert werden. Dabei zeigt Fig. 1 eine perspektivische Prinzipdarstellung eines Konnektionselements 1 nach einer ersten Ausführungsform. Das Konnektionselement 1 weist eine Einlassleitung 2 auf. Über diese Einlassleitung 2 kann Reinwasser aus einer Dialysemaschine in das Konnektionselement 1 eingeleitet werden und über das Konnektionselement 1 in einen an der Dialysemaschine angebrachten Konzentratbeutel eingeleitet werden. Der hier nicht dargestellte Konzentratbeutel kann dabei mit Bicarbonat in Form eines Trockenpulvers gefüllt sein.

Das über das Konnektionselement 1 in den Konzentratbeutel einströmende Reinwasser vermischt sich in dem Konzentratbeutel mit dem Trockenpulver. Anschließend kann die durch das Vermischen entstandene Konzentratlösung aus dem Konnektionselement 1 ausströmen. Hierfür weist das Konnektionselement 1 eine Auslassleitung 3 auf. Die Einlassleitung 2 und die Auslassleitung 3 können dabei parallel zueinander ausgebildet sein und in der gleichen Richtung geöffnet sein. In der Einlassleitung 2 wird das einströmende Fluid umgelenkt. Beispielsweise wie in dieser Ausführungsform gezeigt, wird das Fluid um 90 Grad umgelenkt. Das Konnektionselement 1 weist weiter eine erste Abfuhrleitung 21 auf.

Die erste Abfuhrleitung 21 ist dabei in einem ersten Teilabschnitt 22 um die Einlassleitung 2 ausgebildet. Wie in Fig. 1 dargestellt, kann die Abfuhrleitung 21 ein Hohlzylinder sein, in welchem sich die Einlassleitung 2 teilweise erstreckt. Wird das Konnektionselement 1, wie in Fig. 2 gezeigt, in eine Reinigungsvorrichtung 8 eingefahren, das heißt ist nicht mit einem Konzentratbeutel verbunden, kann durch die erste Abfuhrleitung 21 eine Reinigung der Einlassleitung 2 stattfinden. Fig. 2 zeigt dabei eine Schnittansicht des Konnektionselements 1 nach einer zweiten Ausführungsform. Eine Reinigung des Konnektionselements 1 wird beispielsweise nach einer Dialysebehandlung durchgeführt. Hierbei wird der gesamte Dialysatkreislauf durchgespült, wobei hier Desinfektionsmittel in den Dialysatkreislauf eingeleitet wird. Ist das Konnektionselement 1 zur Reinigung in die Reinigungsvorrichtung 8 eingefahren, kann das Fluid, jetzt Desinfektionsmittel, in der Reinigungsvorrichtung 8 umgeleitet werden und durch die erste Abfuhrleitung 21 aus dem Konnektionselement 1 ausströmen. Der Fluidfluss ist in Fig. 2 dargestellt. Dabei umspült das Desinfektionsmittel die Einlassleitung 2, da das Desinfektionsmittel über die Abfuhrleitung abgeführt wird. Für in der Reinigungsvorrichtung 8 verbleibende Restflüssigkeit kann in

Da die erste Abfuhrleitung 21 sich um den Außenumfang der Einlassleitung 2 erstreckt, kann sichergestellt werden, dass diese ausreichend desinfiziert wird. Auch um die Auslassleitung 3 ist analog eine zweite Abfuhrleitung 31 ausgebildet. Die erste und zweite Abfuhrleitung 31 können sich, wie in Fig. 1 gezeigt, weiter erstrecken als die Einlassleitung 2, beziehungsweise Auslassleitung 3 erstrecken. Mit anderen Worten kann das Ende der ersten und zweiten Abfuhrleitung 31 mit der Reinigungsvorrichtung 8, beispielsweise mit deren Bodenfläche, in Kontakt stehen, das Ende der Einlassleitung 2, beziehungsweise Auslassleitung 3, aber von der Bodenfläche der Reinigungsvorrichtung 8 beabstandet ausgebildet sein.

Mit anderen Worten ist auch bei Einbringen des Konnektionselements 1 in der Reinigungsvorrichtung 8 mit ebener Bodenfläche eine fluidische Verbindung zwischen erster Abfuhrleitung 21 und Einlassleitung 2, beziehungsweise zweiter Abfuhrleitung 31 und Auslassleitung 3 möglich. Wie in Fig. 1 dargestellt weist das Konnektionselement 1 weiter einen Mittelabschnitt 4 auf. Von einem Ende des Mittelabschnitt 4 erstrecken sich die Einlassleitung 2 und die erste Abfuhrleitung 21 parallel in jeweils zwei voneinander beabstandeten zylinderförmigen Vorsprüngen 61, 62. Die diesen Vorsprüngen 61, 62 fluidisch entgegengesetzten anderen Enden der Einlassleitung 2 und ersten Abfuhrleitung 21 erstrecken sich im Wesentlichen senkrecht zu diesen Vorsprüngen 61, 62 von dem Mittelabschnitt 4 in einem zylinderförmigen ersten Fortsatz 51.

In entsprechender Weise sind die Auslassleitung 3 und zweite Abfuhrleitung 31 an einem zweiten Ende des Mittelabschnitts 4 angeordnet. Das Konnektionselement 1 kann, wie in Fig. 3 gezeigt, Führungselemente 41 aufweisen. Die Führungselemente 41 können dabei integral mit dem Konnektionselement 1 ausgebildet sein. Alternativ können die Führungselemente 41 wie in dieser Ausführungsform gezeigt, weitere Bauelemente sein, welche an dem Konnektionselement 1 anbringbar sind. Hierfür weisen die Führungselemente 41 Durchgangsbohrungen mit Innengewinde und das Konnektionselement 1 an dem ersten und zweiten Ende Sackbohrungen mit Innengewinde auf. Hierdurch kann das Führungselement 41 an dem Konnektionselement 1 befestigt werden. Alternativ kann das Führungselement 41 mit dem Konnektionselement 1 verklebt, vernietet oder verschweißt werden.

Die Führungselemente 41 können das Konnektionselement 1 wie später gezeigt werden wird, mit einer in einem Gehäuse 7 angeordneten Führungsstruktur 71 bewegbar anordnen. Das Konnektionselement 1 kann weiter ein Positionierungselement aufweisen. Das Positionierungselement 42 ist in der in Fig. 3 gezeigten Ausführungsform ein sich von dem Mittelelement erstreckender Vorsprung. Mit Hilfe dieses Positionierungselements kann die Position des Konnektionselements 1 erkannt werden, indem das Konnektionselement 1 mit einer dem Gehäuse 7 zugeordneten Lichtschranke wechselwirkt.

Fig. 3 zeigt eine Teilansicht des Konnektionssystems nach einer Ausführungsform eines Konnektionssystems. Das Konnektionssystem kann ein Konnektionselement 1 nach der zuvor beschriebenen Ausführungsform aufweisen. Weiter weist das Konnektionssystem ein Gehäuse 7 auf. In dem Gehäuse 7 sind Konnektionsführungen 71 angeordnet, welche wie in Fig. 3 gezeigt, als zwei von der Gehäuseinnenwand hervorstehende Linearführungen ausgebildet sind. Über das zuvor beschriebene an dem Konnektionselement 1 ausgebildete Führungselement 41 kann das Konnektionselement 1 linear in dem Gehäuse 7 verfahren werden. Das Konnektionssystem weist weiter eine Antriebseinheit 43 auf. Die Antriebseinheit 43 zum Antreiben des Konnektionselement 1 ist in dieser Ausführungsform außerhalb des Gehäuses 7 angeordnet. Alternativ kann die Antriebseinheit 43 aber auch in dem Gehäuse 7 angeordnet sein.

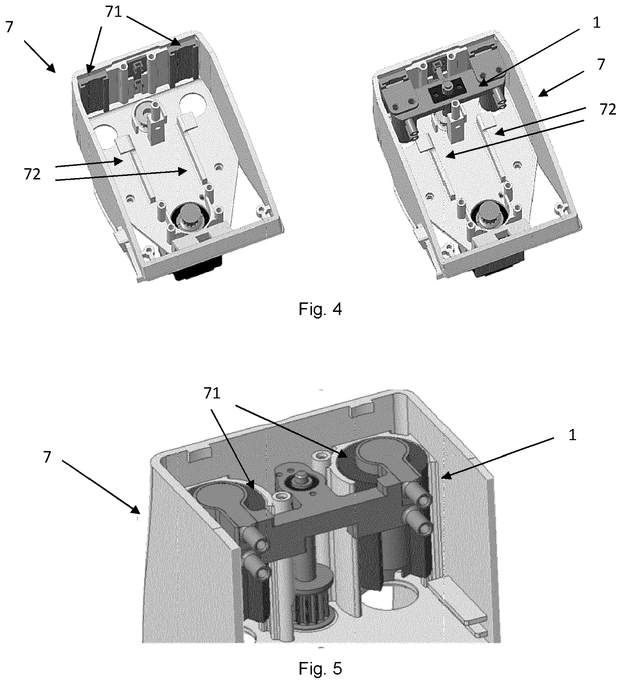

Die Antriebseinheit 43 ist hier ein Elektromotor. In dem Mittelabschnitt 4 des Konnektionselements 1 ist eine Gewindemutter angeordnet. In dem Gehäuse 7 ist weiter eine Gewindespindel, die mit der Gewindemutter des Konnektionselements 1 in Eingriff steht, angeordnet. Die Gewindespindel wird durch den Elektromotor angetrieben. Hierfür ist um die Gewindespindel eine Zahnriemenscheibe, die angetriebene Zahnriemenscheibe, angeordnet, beispielsweise in Presspassung. Um die Zahnriemenscheibe ist ein Zahnriemen gelegt, über welchen die Kraftübertragung von der mit dem Elektromotor angetriebenen Zahnriemenscheibe erfolgt. Fig. 4 zeigt die Führung des Konnektionselements 1 in den Konnektionsführungen 71. Dabei sind Kugelführungsbuchsen in dem Gehäuse 7 angeordnet, in welchen jeweils die zylinderförmigen ersten und zweiten Fortsätze 51, 52 verschiebbar angeordnet werden. Alternativ können Führungsschienen 71, wie in der Ausführungsform in Fig. 5 gezeigt, beispielsweise aus Aluminium, verwendet werden, wobei hier ein Führungsschlitten, beispielsweise aus Kunststoff an dem Konnektionselement 1 angebracht werden kann.

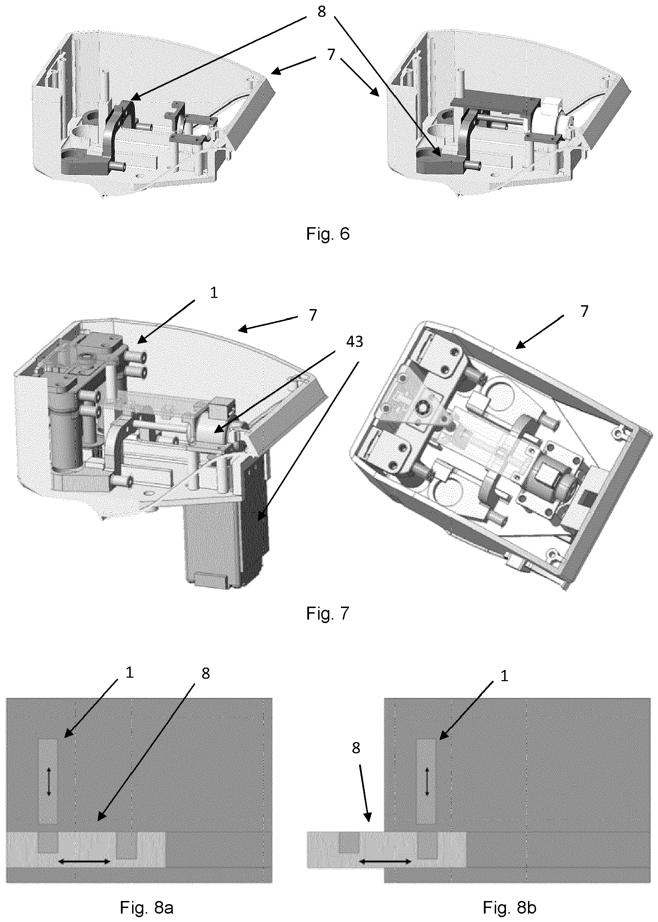

In dem Gehäuse 7 ist weiter eine Reinigungsvorrichtung 8 verfahrbar, wie in Fig. 6 gezeigt, angeordnet. Hierfür sind in dem Gehäuse 7 Reinigungsführungen 72 senkrecht zu den Konnektionsführungen 71 ausgebildet. Zum Antreiben der Reinigungsvorrichtung 8 kann eine zweite Antriebseinheit 43 in dem Gehäuse 7 angeordnet sein. Die durch den Elektromotor, wie in Fig. 7b gezeigt, angetriebene Gewindespindel greift in eine an der Reinigungsvorrichtung 8 ausgebildete Gewindemutter ein.

Fig. 7 zeigt eine perspektivische Ansicht des Konnektionssystems nach der ersten Ausführungsform, insbesondere die Anordnung des Konnektionssystems. Zum Ansteuern der Antriebseinheit 43 des Konnektionselements 1 und Reinigungsvorrichtung 8 kann eine Steuereinheit in dem Gehäuse 7 angeordnet oder diesem zugeordnet sein. Hierdurch sind das Konnektionselement 1 zwischen mehreren, vorzugsweise drei Positionen, verfahrbar. Die Bestimmung der Position kann über Sensoren erfolgen und ist in der hier gezeigten Ausführungsform durch Lichtschranken in dem Gehäuse 7, welche das Positionierungselement des Konnektionselements 1 erfassen können, verwirklicht. Auch die Reinigungsvorrichtung 8 kann zwischen mehreren, vorzugsweise zwei, Positionen verfahren werden. Auch die Positionsbestimmung der Reinigungsvorrichtung 8 kann über Lichtschranken erfolgen.

In Fig. 8a und 8b ist eine Prinzipdarstellung von Freiheitsgraden eines Konnektionsverfahrens nach einer ersten Ausführungsform gezeigt. In Fig. 8a ist ein Zustand zur Erreichung einer Konnektionsstellung gezeigt. In dieser Position kann das Konnektionselement 1 vertikal nach unten verfahren werden, um so in die Reinigungsvorrichtung 8 einzugreifen. In der Konnektionsstellung sind die ersten und zweiten Fortsätze des Konnektionselements 1 mit dem Konzentratbeutel verbunden, so dass eine fluidische Verbindung über die Einlassleitung 2, durch den Konzentratbeutel, und über die Auslassleitung 3 zurück zur Dialysemaschine ausbildbar ist. Mit anderen Worten wird in der Konnektionsstellung Reinwasser aus dem hierfür vorgesehenen Anschluss der Dialysemaschine über die Einlassleitung 2 des Konnektionselements 1 in den Konzentratbeutel eingeführt, mit dem Trockenkonzentrat vermischt, um anschließend über die Auslassleitung 3 des Konnektionselements 1 wieder der Dialysemaschine zugeführt zu werden.

Nach Beendigung der Dialysebehandlung verfährt das Konnektionselement 1 aus der Reinigungsvorrichtung 8 vertikal in eine Warteposition. In dieser Warteposition ist das Konnektionselement 1 nicht mit der Reinigungsvorrichtung 8 in Eingriff. Zur Ausbildung einer Reinigungsstellung verfährt anschließend die Reinigungsvorrichtung 8 horizontal. Der an der in dieser Ausführungsform an der Reinigungsvorrichtung 8 angebrachte Konzentratbeutel wird mit der Reinigungsvorrichtung 8 verfahren, um so entnommen werden zu können. Von der Entnahme des Konzentratbeutels unabhängig kann das Konnektionselement 1 vertikal in eine Reinigungsstellung in das Reinigungselement 8 verfahren. In der Reinigungsstellung sind die ersten und zweiten Fortsätze in Leitungselementen, welche als Sacklöcher in der Reinigungsvorrichtung 8 ausgebildet sein können, in Eingriff.

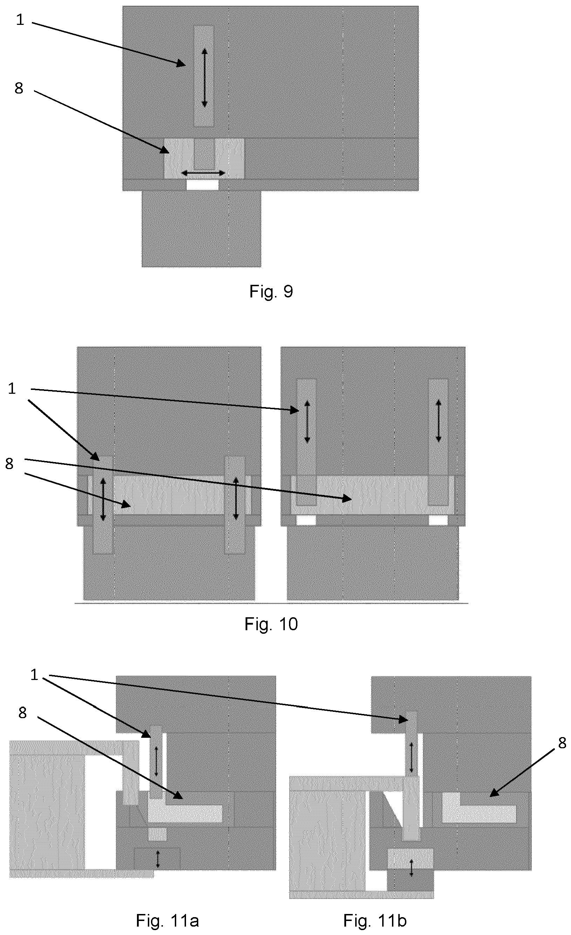

In der Reinigungsstellung kann damit ein Fluidfluss über die Einlassleitung 2 hinein und die erste Abfuhrleitung 21 heraus erreicht werden und analog eine Fluidfluss über die Auslassleitung 3 hinein und die zweite Abfuhrleitung 21 heraus. Hierdurch erfolgt eine Reinigung der Einlassleitung 2 und Auslassleitung 3. In dieser Reinigungsstellung können nun die Einlassleitung 2 und Auslassleitung 3 gereinigt werden. Fig. 9 zeigt eine weitere, zweite, Ausführungsform als Prinzipdarstellung der Bewegungsabläufe des Konnektionsverfahrens. Hierbei ist der Konzentratbeutel nicht an der Reinigungsvorrichtung 8 angebracht um mit dieser Verfahren zu können. Der Konzentratbeutel wird bei dieser Ausführungsform von dem Pflegepersonal an dem Gehäuse 7 angebracht. Zur Erreichung der Konnektionsstellung verfährt die Reinigungsvorrichtung 8 horizontal, so dass das Konnektionselement 1 vertikal nach unten verfahren und so mit dem Konzentratbeutel konnektiert werden kann.

Nach der Dialysebehandlung verfährt das Konnektionselement 1 von dem Konzentratbeutel vertikal nach oben. Der Konzentratbeutel kann nun von dem Pflegepersonal entfernt werden. Davon unabhängig verfährt die Reinigungsvorrichtung 8 horizontal unter das Konnektionselement 1. Anschließend verfährt das Konnektionselement 1 vertikal um in Eingriff mit der Reinigungsvorrichtung 8 zu kommen und so die Reinigungsstellung auszubilden. Fig. 10 zeigt das Konnektionsverfahren nach der zweiten Ausführungsform in einer Vorderansicht. Fig. 11a und 11b zeigen eine weitere Ausführungsform des Konnektionsverfahrens. Fig. 11a zeigt dabei die Reinigungsstellung, in welcher sich das Konnektionselement 1 in der Reinigungsvorrichtung 8 befindet. Während sich das Konnektionselement 1 in der Reinigungsstellung befindet, kann ein Konzentratbeutel an dem Gehäuse 7 angebracht werden. Verfährt das Konnektionselement 1 nach der Reinigung aus der Reinigungsvorrichtung 8 vertikal von dieser weg, kann anschließend die Reinigungsvorrichtung 8 horizontal verfahren.

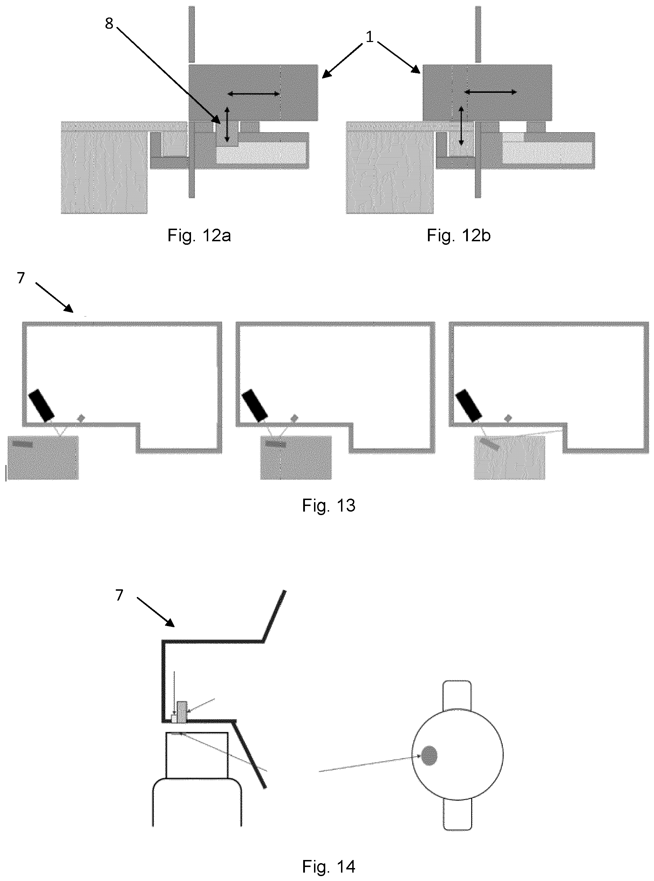

Hierdurch wird die Position unterhalb des Konnektionselements 1 frei, wodurch der Konzentratbeutel, beispielsweise begünstigt durch eine schräge Zuführung und/oder ein Federelement, verschoben wird. Befindet sich der Konzentratbeutel unterhalb des Konnektionselements 1, verfährt diese anschließend um eine Konnektionsstellung einzunehmen, und damit mit dem Konzentratbeutel verbunden zu sein. Fig. 12a und Fig. 12b zeigen eine weitere Prinzipdarstellung des Bewegungsablaufs des Konnektionsverfahrens. Hierbei ist die Aufnahme für den Konzentratbeutel außerhalb an dem Gehäuse 7 angebracht. Das Konnektionselement 1 kann dagegen aus dem Gehäuse 7 heraus verfahren werden. Die Reinigungsvorrichtung 8 ist unterhalb des Konnektionselements 1 in dem Gehäuse 7 angeordnet. Zur Erreichung der Konnektionsstellung wird das Konnektionselement 1 horizontal aus dem Gehäuse 7 verfahren.

Anschließend kann das Konnektionselement 1 vertikal zum Eingriff in den Konzentratbeutel verfahren werden. Alternativ kann der Konzentratbeutel verschiebbar an dem Gehäuse 7 angeordnet sein, so dass der Konzentratbeutel in Richtung des Konnektionselement 1 verschoben werden kann. Anschließend wird das Konnektionselement 1 wieder horizontal in das Gehäuse 7 zurück verfahren. Um die Reinigungsstellung einzunehmen, kann das Konnektionselement 1 vertikal in die Reinigungsvorrichtung 8 verfahren werden. Alternativ kann die Reinigungsvorrichtung 8 vertikal verfahrbar in dem Gehäuse 7 angeordnet sein. Durch einen Eingriff des Konnektionselement 1 mit der Reinigungsvorrichtung 8 wird so die Reinigungsstellung eingenommen.

Zum Erfassen, ob sich ein Konzentratbeutel an dem Gehäuse 7 befindet, kann das Gehäuse 7 eine Erfassungseinheit aufweisen. Hierfür können, wie in einer Prinzipdarstellung in Fig. 13 dargestellt ist, eine Lichtquelle als Sender sowie ein Lichtempfänger an dem Gehäuse 7 ausgebildet sein. Sender und Empfänger können dabei an einer Unterseite des Gehäuses 7, unter welcher der Konzentratbeutel aufgenommen werden kann, ausgebildet sein. Der Konzentratbeutel weist an einer Oberfläche eine Reflexionsgeometrie auf. Sendet die Lichtquelle einen Lichtstrahl in einer bestimmten Richtung aus, so wird der Lichtimpuls in entsprechender Stärke nur an dem Lichtempfänger aufgenommen, wenn der Konzentratbeutel an einer vorgegebenen Position relativ zu dem Gehäuse 7 angeordnet ist.

Die Reflexionsgeometrie auf dem Konzentratbeutel kann dabei variieren, beispielsweise in Abhängigkeit von dem Trockenkonzentrat, welches sich in dem Konzentratbeutel befindet. Damit wird nicht nur sichergestellt, dass sich der Konzentratbeutel an der richtigen Position befindet, vielmehr wird auch sichergestellt, dass das für eine bestimmte Behandlung vorgesehene Trockenkonzentrat bereitgestellt wurde. Wird festgestellt, dass sich ein falscher Konzentratbeutel oder sich dieser an einer falschen Position befindet, mit andere Worten, die Reflexionsgeometrie nicht durch das Empfangen an der Empfangseinheit detektiert wird, kann ein Behandlungsbeginn verhindert werden. Beispielsweise kann dem Pflegepersonal eine Nachricht, beispielsweise in Form eines Alarms, präsentiert werden.

Das Gehäuse 7 kann in einer weiteren Ausführungsform als Erfassungseinheit einen Farbsensor als Empfänger sowie ein Lichtquelle als Sendeinheit aufweisen. Entsprechend kann, wie in der in Fig. 14 gezeigten Prinzipdarstellung der Konzentratbeutel eine Farbmarkierung aufweisen. Dabei kann je nach farblicher Kodierung erfasst werden, welche Art von Trockenkonzentrat oder welcher Konzentratbeuteltyp vorliegt. Ebenso kann eine vordefinierte Füllmenge, sowie eine korrekte Positionierung, erfasst werden. Auch bei dieser Ausführungsform können Lichtquelle sowie Farbsensor an einer Unterseite des Gehäuses 7 ausgebildet sein. Als weitere Ausführungsform kann ein Radarsensor an dem Gehäuse 7 angeordnet sein, und die Lage sowie das Vorhandensein des Konzentratbeutels erfasst werden.

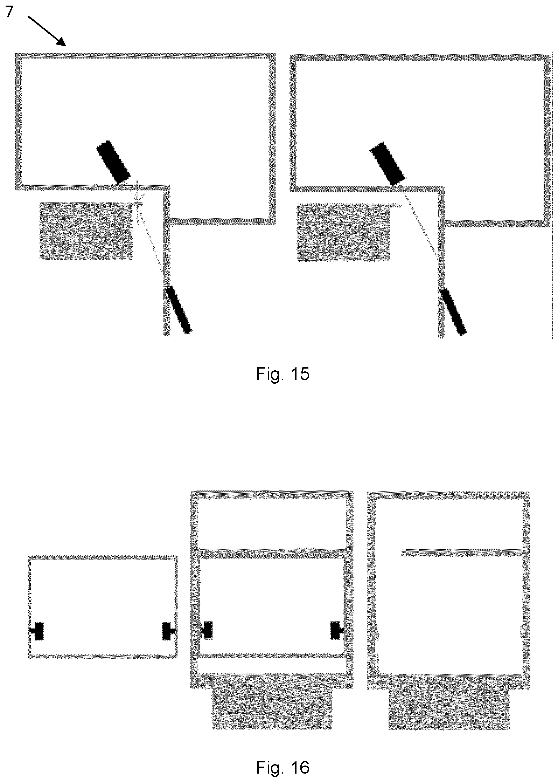

Fig. 15 zeigt eine Prinzipdarstellung einer weiteren Ausführungsform der Erfassungseinheit. Auch bei dieser Ausführungsform weist das Gehäuse 7 ein Sende sowie Empfangseinheit auf. Die Sendeeinheit ist hier eine Lichtquelle. Die Empfangseinheit ist derart an dem Gehäuse 7 angeordnet, dass sie nur einen Lichtimpuls empfängt, wenn sich der richtige Konzentratbeutel an der richtigen Stelle befindet. Dies wird aufgrund der charakteristischen Brechzahl des verwendeten Materials sichergestellt. Dabei wird die Brechzahl des Konzentratbeutels als Grundlage für den erwarteten Reflexionswinkel genommen. Wird an einer entsprechend positionierten Empfangseinheit kein zuvor definierter Lichtimpuls empfangen, sind die zuvor genannten Bedingungen des richtigen Konzentratbeutels und/oder Position desselben nicht erfüllt. Die Empfangseinheit kann dabei, wie in Fig. 15 dargestellt, an einer der Unterseite des Gehäuses 7 in einem Winkel gegenüberliegenden Seite des Gehäuses 7 ausgebildet sein.

Fig. 16 zeigt eine weitere Ausführungsform der Erfassungseinheit. Hierbei weist das Gehäuse 7 an einer Position, an welcher sich der Konzentratbeutel in einer korrekten Position befindet, zumindest einen, vorzugsweise aber mehrere Mikrotaster auf. Wie in Fig. 16 gezeigt, kann der Konzentratbeutel zwei Vorsprünge aufweisen. Der Mikrotaster an den Seitenflächen des Gehäuses 7 kann direkt hinter einer Elastomerschicht angeordnet werden, so dass bei einem Eindrücken dieser durch die Vorsprünge der Mikrotaster ausgelöst wird. Die Kodierung verschiedener Konzentratbeutel kann dabei über die Position der Mikrotaster an dem Gehäuse 7 erfolgen.

## Patentansprüche

1. Konnektionselement (1) für eine Blutbehandlungsvorrichtung, insbesondere eine Dialysemaschine, aufweisend,
eine Einlassleitung (2), zum Einlassen eines Fluids in das Konnektionselement (1),
eine Auslassleitung (3), zum Auslassen eines Fluids aus dem Konnektionselement (1),
zumindest eine erste Abfuhrleitung (21), zum Abführen eines Fluids aus dem Konnektionselement (1),
**dadurch gekennzeichnet, dass**
die erste Abfuhrleitung (21) in einem Teilabschnitt (22) um die Einlassleitung (2) herum ausgebildet ist, und wobei
die Einlassleitung (2) und die erste Abfuhrleitung (21) zu einem ersten Endabschnitt (23) des Konnektionselements (1) geöffnet sind und der erste Endabschnitt (23) fluidisch mit dem Teilabschnitt (22) verbunden ist.

2. Konnektionselement (1) nach Anspruch 1, weiter aufweisend
eine zweite Abfuhrleitung (31), zum Abführen eines Fluids aus dem Konnektionselement (1), wobei die zweite Abfuhrleitung (31) in einem zweiten Teilabschnitt um die Auslassleitung (3) herum ausgebildet ist,
und wobei die Auslassleitung (3) und die zweite Abfuhrleitung (31) zu einem zweiten Endabschnitt des Konnektionselement (1) geöffnet sind und der zweite Endabschnitt fluidisch mit dem Teilabschnitt verbunden ist.

3. Konnektionselement (1) nach Anspruch 1 oder 2, wobei
die Einlassleitung (2) und/oder Auslassleitung (3) und/oder die erste und zweite Abfuhrleitung (31) derart in dem Konnektionselement (1) angeordnet sind, dass eine Umlenkung der Fluidrichtung in dem Konnektionselement (1), um vorzugsweise 120 bis 70 Grad, insbesondere um 110 bis 80 Grad, besonders bevorzugt von im Wesentlichen 90 Grad, erreichbar ist.

4. Konnektionselement (1) nach einem der Ansprüche 1 bis 3, weiter aufweisend einen Mittelabschnitt (4),
einen, vorzugsweise zylinderförmigen, ersten Fortsatz (51), in dem die erste Abfuhrleitung (21) um die Einlassleitung (2) ausgebildet ist, welcher sich von einem ersten Ende des Mittelabschnitts (4) in einer ersten Richtung, vorzugsweise im Wesentlichen senkrecht, von dem Mittelabschnitt (4) erstreckt,
einen, vorzugsweise zylinderförmigen, zweiten Fortsatz (52), in dem die zweite Abfuhrleitung (31) um die Auslassleitung (3) ausgebildet ist, welcher sich von einem zweiten, dem ersten gegenüberliegenden, Ende des Mittelabschnitts (4) in der ersten Richtung, vorzugsweise parallel zu dem ersten Fortsatz (51), von dem Mittelabschnitt (4) erstreckt.

5. Konnektionselement (1) nach einem der Ansprüche 1 bis 4, weiter aufweisend
vier, vorzugsweise zueinander parallel verlaufende, zylinderförmige Vorsprünge (61, 62, 63, 64), wobei sich die Einlassleitung (2) und die erste Abfuhrleitung (21) in je einem Vorsprung von dem ersten Ende des Mittelabschnitts (4) in einer zweiten, von der ersten verschiedenen Richtung, erstrecken, und wobei
sich die Auslassleitung (3) und die zweite Abfuhrleitung (31) in je einem Vorsprung von dem zweiten Ende des Mittelabschnitts (4) in der zweiten Richtung erstrecken.

6. Konnektionselement (1) nach einem der Ansprüche 1 bis 5, weiter aufweisend
Führungselemente (41) zur verfahrbaren, vorzugsweise linear verschiebbaren, Verbindung mit einer Führungsvorrichtung.

7. Konnektionselement (1) nach einem der Ansprüche 1 bis 6, weiter aufweisend
ein Positionierungselement (42), welches in einer dritten, von der ersten und zweiten verschiedenen Richtung, von dem Mittelabschnitt (4) hervorsteht.

8. Konnektionsystem für eine Blutbehandlungsvorrichtung, insbesondere eine Dialysemaschine, aufweisend
ein Konnektionselement (1) nach einem der Ansprüche 1 bis 7,
ein Gehäuse (7), in welchem das Konnektionselement (1) bewegbar angeordnet ist,
ein Reinigungselement (8), welches in dem Gehäuse (7) bewegbar angeordnet ist,
zumindest eine erste Antriebseinheit (43), zum Verfahren des Konnektionselements (1) und/oder des Reinigungselements (8), sowie
eine Steuereinheit zur Ansteuerung der zumindest ersten Antriebseinheit (43).

9. Konnektionssystem nach Anspruch 8, weiter aufweisend,
Konnektionsführungen (71), welche in dem Gehäuse (7) angeordnet sind, auf welchen das Konnektionselement (1) zwischen mehreren, vorzugsweise drei, Positionen verfahrbar ist,
Reinigungsführungen (72), welche in dem Gehäuse (7) angeordnet sind, auf welchen das Reinigungselement (8) zwischen mehreren, vorzugsweise zwei, Positionen verfahrbar ist.

10. Konnektionssystem nach Anspruch 8 oder 9, weiter aufweisend
zumindest einen Positionssensor in dem Gehäuse (7), vorzugsweise eine Lichtschranke, mittels welcher die Position des Konnektionselements (1) und/oder des Reinigungselements (8) erfassbar ist.

11. Konnektionsverfahren zur Konnektion eines Trockenkonzentratbehälters,
wobei ein Gehäuse (7) mit einem in dem Gehäuse (7) auf einer ersten Geraden verfahrbaren Konnektionselement (1) nach einem der Ansprüche 1 bis 7, welches zwei Fortsätze aufweist, und einem in dem Gehäuse (7) auf einer zweiten, zu der ersten Geraden, vorzugsweise im Wesentlichen senkrechten, Geraden, verfahrbaren Reinigungselement (8), in welchem ein erstes und ein zweites Leitungselement ausgebildet sind, ausgebildet ist, die Schritte aufweisend
Verfahren des Konnektionselements (1) auf der ersten Geraden zur Ausbildung einer Reinigungsstellung, wobei in der Reinigungsstellung der erste und zweite Fortsatz (51, 52) des Konnektionselement (1) in Eingriff mit dem ersten und zweiten Leitungselement des Reinigungselements (8) sind.

12. Konnektionsverfahren nach Anspruch 11, weiter aufweisend
Verfahren des Konnektionselements (1) auf der ersten Geraden,
Verfahren der Reinigungsvorrichtung (8) auf der zweiten Geraden,
Verfahren des Konnektionselements (1) auf der ersten Geraden zur Ausbildung der Konnektionsstellung, wobei in dieser Konnektionsstellung, der erste und zweite Fortsatz (51, 52) aus dem Gehäuse (7) hervorstehen.

13. Konnektionsverfahren nach einem der Ansprüche 11 oder 12, weiter aufweisend
Anbringen eines Behälters, vorzugsweise mit Trockenkonzentrat gefüllt, an dem Gehäuse (7), wenn das Konnektionselements (1) in der Reinigungsstellung ist,
Erkennen, ob der Behälter zur Konnektion bereitgestellt ist,
Verfahren des Konnektionselements (1) auf der ersten Geraden in die Konnektionsstellung,
Verbinden des Behälters mit dem ersten und zweiten Fortsatz (51, 52) des Konnektionselements (1), so dass eine fluidische Verbindung hergestellt wird.

14. Konnektionsverfahren nach einem der Ansprüche 11 bis 13, wobei das Konnektionselement (1) fluidisch mit einer Blutbehandlungsvorrichtung verbindbar ist, weiter aufweisend
Verfahren des Konnektionselements (1) auf der ersten Geraden in einer ersten Richtung,
Verfahren des Reinigungselements (8) auf der zweiten Geraden,
Verfahren des Konnektionselements (1) auf der ersten Geraden in einer der ersten Richtung entgegengesetzten Richtung zur Ausbildung der Reinigungsstellung, so dass der erste und zweite Fortsatz (51, 52) jeweils in dem ersten und zweiten Leitungselement der Reinigungsvorrichtung (8) aufgenommen sind,
Reinigen des ersten und zweiten Fortsatzes (51, 52) in dem ersten und zweiten Leitungselements.

15. Verwendung eines Konnektionselements (1) nach Anspruch 1 bis 7 bei einer Blutbehandlungsvorrichtung.

## Claims

1. Connection element (1) for a blood treatment device, in particular a dialysis machine, comprising
an inlet line (2), for admitting a fluid into the connection element (1),
an outlet line (3), for removing a fluid from the connection element (1),
at least a first discharge line (21), for discharging a fluid from the connection element (1),
**characterized in that**
the first discharge line (21) is formed in a subsection (22) around the inlet line (2), and wherein
the inlet line (2) and the first discharge line (21) are open to a first end section (23) of the connection element (1), and the first end section (23) is fluidically connected to the subsection (22).

2. Connection element (1) according to Claim 1, further comprising
a second discharge line (31), for discharging a fluid from the connection element (1), wherein the second discharge line (31) is formed in a second subsection around the outlet line (3),
and wherein the outlet line (3) and the second discharge line (31) are open to a second end section of the connection element (1), and the second end section is fluidically connected to the subsection.

3. Connection element (1) according to Claim 1 or 2, wherein
the inlet line (2) and/or outlet line (3) and/or the first and the second discharge line (31) are arranged in the connection element (1) in such a way that a deflection of the fluid direction in the connection element (1) is achievable, by preferably 120 to 70 degrees, in particular by 110 to 80 degrees, particularly preferably by substantially 90 degrees.

4. Connection element (1) according to one of Claims 1 to 3, further comprising a middle section (4),
a preferably cylindrical first extension (51), in which the first discharge line (21) is formed around the inlet line (2) and which extends from a first end of the middle section (4) in a first direction, preferably substantially perpendicularly, from the middle section (4),
a preferably cylindrical second extension (52), in which the second discharge line (31) is formed around the outlet line (3) and which extends from a second end of the middle section (4), opposite from the first end, in the first direction, preferably parallel to the first extension (51).

5. Connection element (1) according to one of Claims 1 to 4, further comprising
four cylindrical projections (61, 62, 63, 64), preferably extending parallel to one another, wherein the inlet line (2) and the first discharge line (21) each extend in a projection from the first end of the middle section (4) in a second direction, different from the first direction, and wherein the outlet line (3) and the second discharge line (31) each extend in a projection from the second end of the middle section (4) in the second direction.

6. Connection element (1) according to one of Claims 1 to 5, further comprising
guide elements (41) for movable, preferably linearly movable, connection to a guide device.

7. Connection element (1) according to one of Claims 1 to 6, further comprising
a positioning element (42), which projects from the middle section (4) in a third direction, different from the first and second directions.

8. Connection system for a blood treatment device, in particular a dialysis machine, comprising
a connection element (1) according to one of Claims 1 to 7,
a housing (7), in which the connection element (1) is movably arranged,
a cleaning element (8), which is arranged movably in the housing (7),
at least a first drive unit (43), for moving the connection element (1) and/or the cleaning element (8), and
a control unit for activating the at least first drive unit (43).

9. Connection system according to Claim 8, further comprising
connection guides (71), which are arranged in the housing (7) and on which the connection element (1) is movable between several positions, preferably three positions,
cleaning guides (72), which are arranged in the housing (7) and on which the cleaning element (8) is movable between several positions, preferably two positions.

10. Connection system according to Claim 8 or 9, further comprising
at least one position sensor in the housing (7), preferably a light barrier by means of which the position of the connection element (1) and/or of the cleaning element (8) is detectable.

11. Connection method for the connection of a dry concentrate container,
wherein a housing (7) is formed with a connection element (1) according to one of Claims 1 to 7 movable in the housing on a first straight line, which connection element has two extensions, and with a cleaning element (8), which is movable in the housing (7) on a second straight line, preferably substantially perpendicular to the first straight line, and in which a first and a second line element are formed, said method comprising the steps of moving the connection element (1) on the first straight line for the formation of a cleaning position, wherein in the cleaning position the first and second extensions (51, 52) of the connection element (1) are in engagement with the first and second line elements of the cleaning element (8).

12. Connection method according to Claim 11, further comprising
moving the connection element (1) on the first straight line,
moving the cleaning device (8) on the second straight line,
moving the connection element (1) on the first straight line for the formation of the connection position, wherein in this connection position the first and second extensions (51, 52) project from the housing (7).

13. Connection method according to either of Claims 11 and 12, further comprising
mounting a container, preferably filled with dry concentrate, on the housing (7) when the connection element (1) is in the cleaning position,
detecting whether the container is provided for connection,
moving the connection element (1) on the first straight line into the connection position,
connecting the container to the first and second extensions (51, 52) of the connection element (1), such that a fluidic connection is produced.

14. Connection method according to one of Claims 11 to 13, wherein the connection element (1) is fluidically connectable to a blood treatment device, further comprising
moving the connection element (1) on the first straight line in a first direction,
moving the cleaning element (8) on the second straight line,
moving the connection element (1) on the first straight line in a direction counter to the first direction, for formation of the cleaning position, such that the first and second extensions (51, 52) are received respectively in the first and second line elements of the cleaning device (8),
cleaning the first and second extensions (51, 52) in the first and second line elements.

15. Use of a connection element (1) according to Claims 1 to 7 in a blood treatment device.

## Revendications

1. Élément de connexion (1) pour un dispositif de traitement du sang, en particulier une machine de dialyse, comprenant
un conduit d'entrée (2) pour l'admission d'un fluide dans l'élément de connexion (1),
un conduit de sortie (3) pour évacuer un fluide de l'élément de connexion (1),
au moins un premier conduit d'évacuation (21) pour évacuer un fluide de l'élément de connexion (1), **caractérisé en ce que**
le premier conduit d'évacuation (21) est formé dans une sous-section (22) autour du conduit d'entrée (2), et dans lequel
le conduit d'entrée (2) et le premier conduit d'évacuation (21) sont ouverts vers une première section d'extrémité (23) de l'élément de connexion (1) et la première section d'extrémité (23) est en communication fluidique avec la sous-section (22).

2. Élément de connexion (1) selon la revendication 1, présentant en outre
un deuxième conduit d'évacuation (31), pour évacuer un fluide de l'élément de connexion (1), le deuxième conduit d'évacuation (31) étant formé dans une deuxième sous-section autour du conduit de sortie (3),
et dans lequel le conduit de sortie (3) et le deuxième conduit d'évacuation (31) sont ouverts vers une deuxième section d'extrémité de l'élément de connexion (1) et la deuxième section d'extrémité est en communication fluidique avec la sous-section.

3. Élément de connexion (1) selon la revendication 1 ou la revendication 2, dans lequel
le conduit d'entrée (2) et/ou le conduit de sortie (3) et/ou le premier et le deuxième conduit d'évacuation (31) sont agencés dans l'élément de connexion (1) de telle sorte qu'une déviation de la direction du fluide dans l'élément de connexion (1) est apte à être obtenue, de préférence de 120 à 70 degrés, en particulier de 110 à 80 degrés, de manière particulièrement préférée d'essentiellement 90 degrés.

4. Élément de connexion (1) selon l'une des revendications 1 à 3, comprenant en outre une section centrale (4),
une première extension (51), de préférence cylindrique, dans laquelle le premier conduit d'évacuation (21) est formé autour du conduit d'entrée (2), qui s'étend depuis une première extrémité de la section centrale (4) dans une première direction, de préférence sensiblement perpendiculaire à la section centrale (4),
une deuxième extension (52), de préférence cylindrique, dans laquelle le deuxième conduit d'évacuation (31) est formé autour du conduit de sortie (3), qui s'étend depuis une deuxième extrémité, opposée à la première, de la section centrale (4) dans la première direction, de préférence parallèlement au première extension (51), depuis la section centrale (4).

5. Élément de connexion (1) selon l'une des revendications 1 à 4, comprenant en outre
quatre saillies cylindriques (61, 62, 63, 64), de préférence parallèles entre elles, le conduit d'entrée (2) et le premier conduit de sortie (21) s'étendant chacun en saillie depuis la première extrémité de la partie centrale (4) dans une deuxième direction différente de la première, et dans lequel
le conduit de sortie (3) et le deuxième conduit d'évacuation (31) s'étendent chacun en saillie à partir de la deuxième extrémité de la section centrale (4) dans la deuxième direction.

6. Élément de connexion (1) selon l'une des revendications 1 à 5, comprenant en outre
des éléments de guidage (41) pour la connexion mobile, de préférence à mouvement linéaire, avec un dispositif de guidage.

7. Élément de connexion (1) selon l'une des revendications 1 à 6, présentant en outre
un élément de positionnement (42) faisant saillie de la partie centrale (4) dans une troisième direction, différente de la première et de la deuxième direction.

8. Système de connexion pour un dispositif de traitement du sang, en particulier une machine de dialyse, comprenant
un élément de connexion (1) selon l'une des revendications 1 à 7,
un boîtier (7) dans lequel l'élément de connexion (1) est disposé de manière mobile,
un élément de nettoyage (8), qui est disposé de manière mobile dans le boîtier (7),
au moins une première unité d'entraînement (43) pour déplacer l'élément de connexion (1) et/ou l'élément de nettoyage (8), ainsi que
une unité de commande pour commander ladite au moins une première unité d'entraînement (43).

9. Système de connexion selon la revendication 8, comprenant en outre,
des guides de connexion (71) qui sont agencés dans le boîtier (7) et sur lesquels l'élément de connexion (1) est apte à être déplacé entre plusieurs positions, de préférence trois positions,
des guides de nettoyage (72), qui sont agencés dans le boîtier (7) et sur lesquels l'élément de nettoyage (8) est apte à être déplacé entre plusieurs positions, de préférence deux positions.

10. Système de connexion selon la revendication 8 ou la revendication 9, comprenant en outre
au moins un capteur de position dans le boîtier (7), de préférence une barrière lumineuse, au moyen duquel la position de l'élément de connexion (1) et/ou de l'élément de nettoyage (8) peut être détectée.

11. Procédé de connexion pour la connexion d'un récipient de concentré sec, dans lequel un boîtier (7) ayant un élément de connexion (1) selon l'une des revendications 1 à 7, déplaçable dans le boîtier (7) sur une première ligne droite, qui présente deux extensions, et un élément de nettoyage (8) déplaçable dans le boîtier (7) sur une deuxième ligne droite, de préférence sensiblement perpendiculaire à la première ligne droite, dans lequel sont formés un premier et un deuxième élément de conduite, comprenant les étapes
de déplacement de l'élément de connexion (1) sur la première ligne droite pour former une position de nettoyage, les première et deuxième extensions (51, 52) de l'élément de connexion (1) étant, dans la position de nettoyage, en engagement avec les premier et deuxième éléments de conduit de l'élément de nettoyage (8).

12. Procédé de connexion selon la revendication 11, comprenant en outre
le déplacement de l'élément de connexion (1) sur la première ligne droite,
le déplacement de l'élément de nettoyage (8) sur la deuxième ligne droite,
le déplacement de l'élément de connexion (1) sur la première ligne droite pour former la position de connexion, la première et la deuxième extensions (51, 52) dépassant du boîtier (7) dans cette position de connexion,.

13. Procédé de connexion selon l'une des revendications 11 ou 12, comprenant en outre
la mise en place d'un récipient, de préférence rempli de concentré sec, sur le boîtier (7) lorsque l'élément de connexion (1) est dans la position de nettoyage,
la détection du fait que le récipient est ou non prêt pour la connexion,
le déplacement de l'élément de connexion (1) sur la première ligne droite vers la position de connexion,
la connexion du récipient aux première et deuxième extensions (51, 52) de l'élément de connexion (1) de manière à établir une connexion fluidique.

14. Procédé de connexion selon l'une des revendications 11 à 13, dans lequel l'élément de connexion (1) est apte à être connecté fluidiquement à un dispositif de traitement du sang, comprenant en outre le déplacement de l'élément de connexion (1) sur la première ligne droite dans une première direction,
le déplacement de l'élément de nettoyage (8) sur la deuxième ligne droite,
le déplacement de l'élément de connexion (1) sur la première ligne droite dans une direction opposée à la première direction pour former la position de nettoyage, de sorte que les première et deuxième extensions (51, 52) soient chacune reçues dans les premier et deuxième éléments de conduit du dispositif de nettoyage (8),
le nettoyage des premier et deuxième extensions (51, 52) dans les premier et deuxième éléments de conduit.

15. Utilisation d'un élément de connexion (1) selon les revendications 1 à 7 dans un dispositif de traitement du sang.
